# EUROPEAN PATENT APPLICATION

(11) **EP 1 862 172 A1**
(43) Date of publication of application: **05.12.2007**
(21) Application number: 06076133.5
(22) Date of filing: 31.05.2006
(51) Int. Cl.: A61K 38/01, A61K 31/198, A61K 31/197, A61P 13/12

(54) **Combination of amino acid solution and a gelatin for inhibiting renal uptake**

(71) Applicant: BioSynthema Inc., 1871 CZ Schoorl (NL)
(72) Inventor: The designation of the inventor has not yet been filed

(57) **Abstract**

The invention relates to a method of inhibiting renal uptake of substances, that are potentially damaging for the kidneys, in a living being, by co-administering a composition comprising at least one amino acid, if desired in the form of a pharmaceutically acceptable salt or carboxylic acid derivative, and in addition a gelatin.

## Description

The present invention relates to a method of inhibiting renal uptake of substances, that are potentially damaging for the kidneys, in a living being, by co-administering a composition comprising at least one amino acid, if desired in the form of a pharmaceutically acceptable salt or carboxylic acid derivative.

Such a method is known from EP 0094378 (PCT/EP00/06917).
Radionuclide labeled peptides and also monoclonal antibodies or their fragments and other compounds like certain antibiotics or chemotherapeutic agents undergo undesired renal uptake and cellular retention leading to a high kidney dose, or concentration- Increased doses of radiation or higher concentrations of toxic substances in the kidneys may eventually lead to kidney damage.

The above patent publication has described that co-administration of non-target substances, like lysine in combination with arginine, can reduce non-target kidney retention of immunoconjugates, metabolites thereof and other substances that are potentially damaging to the kidneys, such as defined above.

Just recently it was published that the gelatin-based plasma-expanders Gelofusine^{R} (B. Braun, Germany) and Haemacel^{R} can cause tubular proteinuria Gelatine based solutions are used in clinical medicine to control blood pressure levels in hemorraghic and septic shock, as well as in the post-surgical situation. Gelofusine^{R} is a synthetic colloidal solution based on bovine bone-derived gelatin, and can be considered as a gelatine-based plasma expander. Haemaccel is a synthetic colloidal solution of urea cross linked degraded gelatin molecules. It recently was published that Gelofusine^{R} could successfully be applied to reduce kidney uptake of radiolabeled octreotide to a level comparable to that of lysine. At present it is not clear by what mechanism gelatin-based plasma expanders inhibit kidney uptake of radiolabeled octreotide.

It has now been found, that the combination of gelatine-based plasma expanders and amino acid solutions, in general, can be useful in reducing the kidney uptake and retention of all radiolabeled peptides that are cleared through the kidneys. More specifically, the combination of Gelofusine^{R} and lysine can be used to reduce kidney retention of [¹⁷⁷Lu-DOTA⁰,Tyr³]octreotate (see the Example attached). This would impact current peptide radionuclide radiotherapy protocols in a way that higher tumor radiation doses can be achieved without harming the kidneys. In addition, this may not only account for combinations of 1-lysine and Gelofusine^{R}, but also for combinations of Gelofusine^{R} and LysArg or commercially available amino acid solutions.
The combinations mentioned may also be used to reduce kidney uptake and retention of other toxic compounds that undergo undesired renal uptake and cellular retention, like monoclonal antibodies or their fragments and other compounds like certain antibiotics or chemotherapeutic agents.

The invention also relates to the use of a gelatin in combination with at least one amino acid for the preparation of a composition for inhibiting renal uptake of substances, that are potentially damaging for the kidneys, in a living being, wherein said at least on amino acid and said gelatin are as defined hereinbefore. In a preferred embodiment of the composition the amount of the amino acid is between 150 and 700 mg per kg body weight of the being, and the amount of the gelatin between 50 and 250 mg per kg body weight of the being.

The invention further relates to a therapeutic composition for the inhibition of renal uptake of substances, that are potentially damaging for the kidneys and that are used for therapeutic or diagnostic purposes, in a living being, which composition comprises one or more pharmaceutically acceptable excipients, carriers and/or diluents and a combination of a gelatin and at least one amino acid as defined hereinbefore.

The invention will now be illustrated by the following specific Example.

### Example

### Reduction of kidney uptake of [¹⁷⁷Lu-DOTA⁰,Tyr³]octreotate by the combination of gelofusine and lysine as visualised by NanoSPECT in rats

Aim: Peptide receptor radionuclide therapy (PRRT) using radiolabeled somatostatin analogs, kidney uptake of radiolabeled compound is the major dose-limiting factor. Positively charged amino acids are extensively used to reduce this uptake and to allow higher doses to be administered to patients. Recently it was shown that the gelatine-based plasma expander Gelofusine^{R} was capable to reduce kidney uptake of diagnostic doses of Octreoscan to a level comparable to that by lysine. We studied the effects of Gelofusine^{R} and lysine in therapeutic setting.

Method: Male Lewis rats (5-6 rats per group) were injected with 555 MBq [¹⁷⁷Lu-DOTA⁰,Tyr³]octreotate. For reduction of kidney uptake rats were injected with 20 mg Gelofusine^{R}, 100 mg lysine or the combination. Kidney uptake was measured by SPECT scans with a four-detector multi-pinhole camera (NanoSPECT, Bioscan) at 24h, 5 and 7 days pi. Kidney uptake was quantified by VOI analysis.

Results: At 24h pi. kidney uptake of [⁷⁷Lu-DOTA⁰,Tyr³]octreotate was significantly reduced by both lysine and Gelofusine^{R} (37%±6% and 43%±18% inhibition, respectively). The combination of Gelofusine^{R} and lysine resulted in 65%±11% inhibition of kidney uptake (P<0.01 vs. lysine alone; P<0.05 vs. Gelofusine^{R} alone). Five and seven days pi. inhibition levels were comparable to those 24h pi.

Conclusion: Rat kidney uptake of radiolabeled somatostatin analogs can be monitored for a longer period in the same animal using animal SPECT. Gelofusine^{R} reduced kidney uptake of therapeutic doses [¹⁷⁷Lu-DOTA⁰,Tyr³]octreotate to a level comparable to that after lysine. The combination of these compounds led to a significantly stronger reduction than lysine alone or Gelofusine^{R} alone. This may offer new possibilities in PRRT.

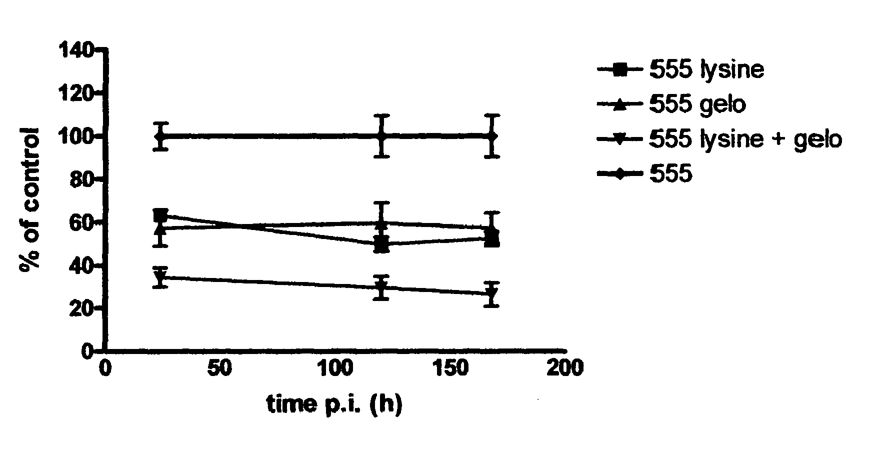

## Claims

1. A method of inhibiting renal uptake of substances, that are potentially damaging for the kidneys, in a living being, by co-administering a composition comprising at least one amino acid, if desired in the form of a pharmaceutically acceptable salt or carboxylic acid derivative, **characterized in that** said composition comprises in addition a gelatin.

2. Method as claimed in claim 1, wherein said at least one amino acid is a lysine, selected from D-lysine, L-lysine and polylysine.

3. Method as claimed in claim 1, wherein said at least one amino acid is a mixture of a lysine and a second amino acid, selected from arginine and ornithine.

4. Method as claimed in any of the preceding claims, wherein the gelatin is a gelatin-based solution.

5. Method as claimed in claim 4, wherein the gelatin-based solution is a gelatin-based plasma expander, in particular Gelofusine^{R}.

6. Method as claimed in claim 4, wherein the gelatin-based solution is a synthetic colloidal solution of urea cross-linked degraded gelatin molecules, in particular Haemacel^{R}.

7. Method as claimed in any of the preceding claims, wherein said composition comprises at least one amino acid in a quantity of 150 to 700 mg per kg body weight of the being, and the gelatin in a quantity of 50 to 250 mg per kg body weight of the being.

8. Use of a gelatin in combination with at least one amino acid for the preparation of a composition for inhibiting renal uptake of substances, that are potentially damaging for the kidneys, in a living being, wherein said at least on amino acid and said gelatin are as defined in any of the preceding claims.

9. A therapeutic composition for the inhibition of renal uptake of substances, that are potentially damaging for the kidneys and that are used for therapeutic or diagnostic purposes, in a living being, which composition comprises one or more pharmaceutically acceptable excipients, carriers and/or diluents and a combination of a gelatin and at least one amino acid as defined in any of the preceding claims 1-7
